# EUROPÄISCHE PATENTANMELDUNG

(11) **EP 1 923 389 A1**
(43) Veröffentlichungstag der Anmeldung: **21.05.2008**
(21) Anmeldenummer: 06123477.9
(22) Anmeldetag: 03.11.2006
(51) Int. Cl.: C07D 313/04, A61K 8/49

(54) **2-Isopropyl-5-methyloxepan und dessen Verwendung als Riechstoff**

(71) Anmelder: Symrise GmbH & Co. KG, 37603 Holzminden (DE)
(72) Erfinder: Nobis, Markus, 3250 Lyss (CH)
(74) Vertreter: Stilkenböhmer, Uwe Michael

(57) **Zusammenfassung**

Beschrieben wird 2-Isopropyl-5-methyloxepan der Formel **A** 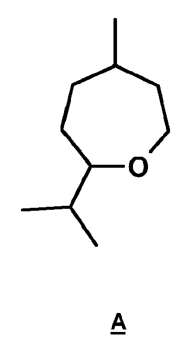 und dessen Verwendung als Riechstoff.

## Beschreibung

Die vorliegende Erfindung betrifft 2-Isopropyl-5-methyloxepan, dessen Verwendung als Riechstoff, entsprechende Verfahren zum Vermitteln, Modifizieren und/oder Verstärken bestimmter Geruchsnoten in einer Riechstoffmischung, entsprechende Riechstoffmischungen und parfümierte Artikel, entsprechende Verfahren zur Herstellung einer Riechstoffmischung sowie Verfahren zur Herstellung von 2-Isopropyl-5-methyloxepan. Weitere Aspekte der Erfindung ergeben sich aus der nachfolgenden Beschreibung sowie den beigefügten Patentansprüchen.

Trotz einer Vielzahl bereits vorhandener Riechstoffe besteht in der Parfümindustrie auch weiterhin ein genereller Bedarf an neuen Riechstoffen mit originellen Geruchseigenschaften, insbesondere an solchen, über Ihre geruchlichen Eigenschaften hinaus zusätzliche positive Sekundäreigenschaften besitzen, wie z. B. eine höhere Ausgiebigkeit, ein besseres Haftungsvermögen, eine geruchlich verstärkende Wirkung in Kombination mit anderen Riechstoffen oder aber auch bessere Ausstrahlung gegenüber vergleichbaren Riechstoffen.

Es besteht insbesondere ein Bedarf an Riechstoffen, die in der Lage sind eine patchouli- und indol-artige Geruchsnote zu erzeugen, insbesondere in Kombination mit anderen Riechstoffen, speziell in Parfümkompositionen.

Der Erfindung liegt insbesondere die überraschende Erkenntnis zugrunde, dass sich die erfindungsgemäße Verbindung 2-Isopropyl-5-methyloxepan als Riechstoff eignet, und zwar ganz besonders überraschend als Riechstoff, der in der Lage ist eine p atchouli- und indol-artige Geruchsnote zu erzeugen. Weitere überraschende Erkenntnisse, die bei der Untersuchung der erfindungsgemäßen Verbindung 2-Isopropyl-5-methyloxepan erkannt wurden, ergeben sich aus der nachfolgenden Beschreibung.

Die Strukturformel von 2-Isopropyl-5-methyloxepan der Formel **A** ist nachfolgend wiedergegeben:

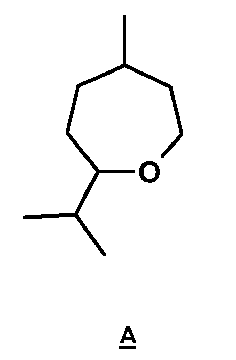

Das erfindungsgemäße 2-Isopropyl-5-methyloxepan der Formel **A** kann dabei cis- oder trans-konfiguriert sein, als (R,R)-konfiguriertes Enantiomer, (R,S)-konfiguriertes Enantiomer, (S,R)-konfiguriertes Enantiomer, (S,S)-konfiguriertes oder als beliebiges Gemisch der Enantiomeren, insbesondere als Racemat, oder auch als beliebiges Gemisch der entsprechenden Diastereomeren, vorliegen.

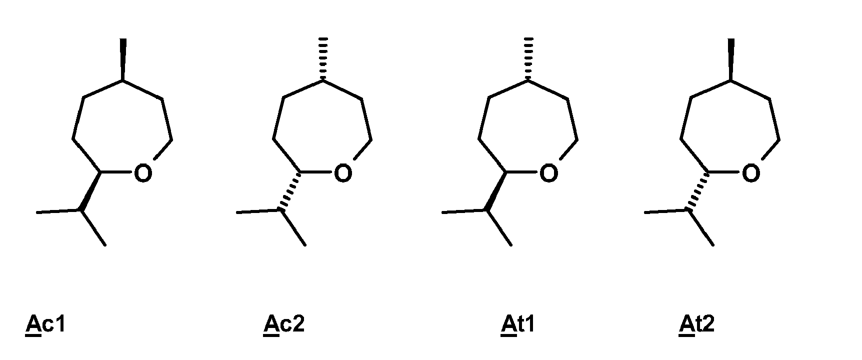

Eine GC-Sniffing Analyse eines cis/trans-Isomerengemisches der Formel **A** zeigte, dass das Geruchsprofil der Isomeren im Wesentlichen identisch ist, wobei das zuerst eluierte Isomer (erster Peak: Retentionsindex 1065, GC-Bedingungen: DB-1, 20 m, 0,18 mm, 60-220°C/9°C pro min.) allerdings im Vergleich zu dem später eluierten Isomer (zweiter Peak: Retentionsindex 1069) den wesentlich stärkeren Geruch aufweist.

Die erfindungsgemäße Verbindung **A** weist nach Meinung der Pafümeure folgendes Geruchsprofil auf: Indol, Kresol, Patchouli, Hyazinthe, Ylang-Ylang, animalisch. Dementsprechend betrifft die vorliegende Erfindung insbesondere die Verwendung von 2-Isopropyl-5-methyloxepan der Formel **A** als Riechstoff mit dem Geruchsprofil Indol, Kresol, Patchouli, Hyazinthe, Ylang-Ylang, animalisch.Die erfindungsgemäße Verbindung **A** zeigt allerdings in hoher Verdünnung einen blumigen Geruch, der an Jasmin und Neroli erinnert. Dementsprechend betrifft die vorliegende Erfindung auch insbesondere die Verwendung von 2-Isopropyl-5-methyloxepan der Formel **A** als Riechstoff in hoher Verdünnung, so dass der Riechstoff einen blumigen Geruch besitzt. Der Fachmann kann die erforderliche hohe Verdünnung z. B. durch Anfertigen einer Verdünnungsreihe schnell erreichen und geeignete Konzentrationsbereiche ermitteln.

Nach Einschätzung der Parfümeure stellt die Verbindung 2-Isopropyl-5-methyloxepan der Formel **A** eine einzigartige Riechstoff-Komponente dar, die aufgrund ihres Charakters vielfältig einsetzbar ist. Insbesondere besitzt die Verbindung **A** ausgeprägte Kopfnoten von Ylang-Ylang und Patchouli, die sie besonders bemerkenswert machen. Im Vergleich zu bislang wirtschaftlich relevanten Riechstoffen mit Patchouligeruch (wie insbesondere Patchoulol, siehe unten) weist Verbindung **A** eine vergleichsweise hohe Flüchtigkeit auf, weshalb sie auch als Bestandteil einer Kopfnote einer Riechstoffmischung wie z. B. eines Parfums eingesetzt werden kann, während die bislang eingesetzten Riechstoffe mit Patchouligeruch eher schwerer flüchtig und in der Regel Bestandteile der Herz- oder Basisnote von Parfüms sind. Die vorliegende Erfindung betrifft dementsprechend insbesondere die Verwendung von 2-Isopropyl-5-methyloxepan der Formel **A** als Riechstoff mit Patchouli- und/oder Ylang-Ylang-Kopfnote.

In eigenen Untersuchungen wurde insbesondere gefunden, dass sich 2-Isopropyl-5-methyloxepan der Formel **A** hervorragend zum Vermitteln, Modifizieren und/oder Verstärken einer, mehrerer oder sämtlicher der Geruchsnoten Indol, Kresol, Patchouli, Hyazinthe, Ylang-Ylang, animalisch eignet. Die Tatsache, dass die Verbindung **A** einen ausdrucksstarken Geruch von Patchouli, Indol und Hyazinthe aufweist, ist besonders überraschend, da **A** sich (a) strukturell deutlich von bekannten Substanzen mit ähnlichen geruchlichen Eigenschaften unterscheidet (Tabelle 1), und (b) die strukturell wohl ähnlichste bekannte Verbindung (Rosenoxid) keinen derartigen Geruch besitzt. Ein erfindungsgemäßes Verfahren zum Vermitteln, Modifizieren und/oder Verstärken einer, mehrerer oder sämtlicher der Geruchsnoten Indol, Kresol, Patchouli, Hyazinthe, Ylang-Ylang, animalisch oder Blume in einer Riechstoffmischung, wobei vorzugsweise zumindest eine, mehrere oder sämtliche der Geruchsnoten Indol, Patchouli, Hyazinthe, Ylang-Ylang oder Blume vermittelt, modifiziert und/oder verstärkt werden, umfasst den folgenden Schritt:
- Mischen der sonstigen Bestandteile der Riechstoffmischung mit einer sensorisch wirksamen Menge an 2-Isopropyl-5-methyloxepan der Formel **A.**

Eine sensorisch wirksame Menge der Verbindung der Formel **A** liegt dabei insbesondere vor, wenn eine, mehrere oder sämtliche der vorgenannten Geruchsnoten in der resultierenden Riechstoffmischung vermittelt, modifiziert und/oder verstärkt werden.

In einem bevorzugten erfindungsgemäßen Verfahren wird eine Patchouli-Kopfnote vermittelt, modifiziert und/oder verstärkt.

In einem alternativen bevorzugten Verfahren wird die Geruchsnote Blume vermittelt, modifiziert und/oder verstärkt; ein solches Verfahren umfasst den folgenden Schritt:
- Mischen der sonstigen Bestandteile der Riechstoffmischung mit einer sensorisch wirksamen Menge an 2-Isopropyl-5-methyloxepan der Formel **A**, so dass das 2-Isopropyl-5-methyloxepan der Formel **A** in der resultierenden Riechstoffmischung in hoher Verdünnung vorliegt.

Die nachfolgende Tabelle 1 zeigt exemplarisch ausgewählte Riechstoffverbindungen und ihre geruchlichen Beschreibungen, die denen der Verbindung **A** zumindest in Aspekten ähneln.

**Tabelle 1:**

| Substanz | Strukturformel | Beschreibung |
|---|---|---|
| Patchoulol | 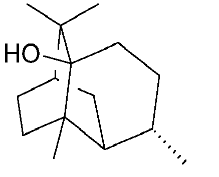 | Starker typisch patchouli-artiger Geruch mit erdiger, leicht campherartiger, pudriger "Keller" Note¹⁾ |
| Indol | 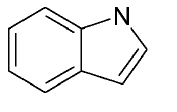 | Unangenehm, fäkalisch, kadaverhaft, in hoher Verdünnung blumig, an Jasmin und Neroli erinnernd ^{2),3)} |
| ω-Bromstyrol | 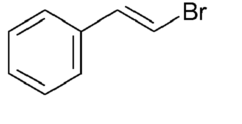 | Hyazinthe, stark ³⁾ |

| | | |
|---|---|---|
| 1) F. Naf, R. Decorzant, W. Giersch & G. Ohloff, A Stereocontrolled Access to (+/-)-, (-)-, and (+)-Patchouli Alcohol, Helv. Chim. Acta, 64, 1387-1397, 1981 2) S. Arctander, Perfume and Flavor Chemicals, Vol I + II, Montclair, N.J. (USA), 1969, Eigenverlag 3) H. Surburg, J. Panten, Common Fragrance and Flavor Materials, 5th ed., Wiley-VCH Verlag GmbH & Co. KG, 2006. | | |

Im Gegensatz zu den Riechstoffen mit den in Tabelle 1 gezeigten Strukturen besitzt die erfindungsgemäße Substanz **A** einen cycloaliphatischen Ring und eine Etherfunktionalität.

Ein bekannter Riechstoff, der 2-Isopropyl-5-methyloxepan der Formel **A** strukturell recht nahe, wenn nicht gar am nächsten kommt, ist Rosenoxid:

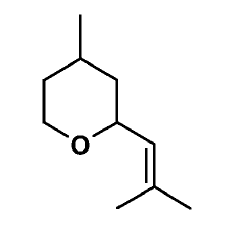

Die sensorischen Eigenschaften von Rosenoxid werden in S. Arctander, Perfume and Flavor Materials, Vol. I und II, Montclair, N. J. 1969, Eigenverlag, als "grasiggrün, rosig und metallisch" und in H. Surburg, J. Panten, Common Fragrance and Flavor Materials, 5th Ed., Wiley-VCH, 2006, als "nach Geranium- und Rosenöl" beschrieben. Somit war es besonders überraschend, dass die erfindungsgemäße Verbindung **A** einen gänzlich anderen, nämlich patchouliartigen und indoligen Geruch aufweist.

Die vorliegende Erfindung betrifft auch Riechstoffmischungen und parfümierte Artikel, die 2-Isopropyl-5-methyloxepan der Formel **A** in einer sensorisch wirksamen Menge umfassen.

Besonders bevorzugt sind erfindungsgemäße Riechstoffmischungen oder parfümierte Artikel, wobei die sensorisch wirksame Menge so gewählt ist, dass das 2-Isopropyl-5-methyloxepan der Formel **A** eine, mehrere oder sämtliche der Geruchsnoten Indol, Kresol, Patchouli, Hyazinthe, Ylang-Ylang, animalisch oder Blume, vorzugsweise zumindest eine, mehrere oder sämtliche der Geruchsnoten Indol, Patchouli, Hyazinthe, Ylang-Ylang oder Blume, vermittelt, modifiziert oder verstärkt. Besonders bevorzugt vermittelt, modifiziert und/oder verstärkt die eingesetzte Menge an 2-Isopropyl-5-methyloxepan der Formel **A** zumindest eine der Geruchsnoten Patchouli oder Indol, vorzugsweise beide.

Besonders bevorzugt sind auch erfindungsgemäße Riechstoffmischungen oder parfümierte Artikel in denen die vorhandene Menge an 2-Isopropyl-5-methyloxepan der Formel **A** eine Patchouli- und/oder Ylang-Ylang-Kopfnote vermittelt, modifiziert oder verstärkt. Alternativ bevorzugt sind erfindungsgemäße Riechstoffmischungen oder parfümierter Artikel, in denen eine derart geringe Menge an 2-Isopropyl-5-methyloxepan der Formel **A** vorliegt, in denen die Verbindung der Formel **A** also so hoch verdünnt ist, dass sie einen blumigen Geruch vermittelt, modifiziert oder verstärkt. Hierbei sind dann häufig weder Patchouli noch Ylang-Ylang-Kopfnoten wahrzunehmen, welche auf die Verbindung **A** zurückgehen würden.

Eine Riechstoffmischung, vorzugsweise eine erfindungsgemäße Riechstoffmischung, wird vorzugsweise nach einem erfindungsgemäßen Verfahren hergestellt, das den folgenden Schritt umfasst:
- Vermischen einer sensorisch wirksamen Menge von 2-Isopropyl-5-methyloxepan der Formel **A** mit weiteren Bestandteilen einer Riechstoffmischung. Eine sensorisch wirksame Menge liegt hierbei insbesondere vor, wenn die eingesetzte menge der Verbindung **A** ausreicht, um in der resultierenden Riechstoffmischung eine, mehrere oder sämtliche Geruchsnoten Indol, Kresol, Patchouli, Hyazinthe, Ylang-Ylang, animalisch oder Blume zu vermitteln, modifizieren oder zu verstärken. Insbesondere liegt eine sensorisch wirksame Menge vor, wenn die eingesetzte Menge der Verbindung **A** ausreicht, um in der Riechstoffmischung eine patchouli- und/oder indol-artige Geruchsnote zu vermitteln, zu modifizieren und/oder zu verstärken (im Vergleich mit einer Mischung der weiteren Bestandteile der resultierenden Riechstoffmischung).

Indolige Geruchsnoten werden in vielfältigen Parfümkompositionen (Riechstoffmischungen) eingesetzt, z. B. in Blumenduft-Themen (d.h. Riechstoffmischungen mit blumigen Noten). Die erfindungsgemäße Verbindung **A** wird daher vorteilhaft Riechstoffmischungen mit bereits vorhandenen blumigen Noten zugesetzt. Das unten folgende Beispiel 1 eines "Jasmin"-Duftthemas" demonstriert in anschaulicher Weise den olfaktorischen Effekt von 2-Isopropyl-5-methyloxepan **A.**

Patchouliöl wird in der Parfümerie z. B. in Riechstoffmischungen mit orientalischen oder maskulinen Noten eingesetzt , ebenso in größerem Ausmaß in Weichspülern. Die erfindungsgemäße Verbindung 2-Isopropyl-5-methyloxepan der Formel **A** wird daher entsprechend vorzugsweise in Riechstoffmischungen mit orientalischen oder maskulinen Noten, sowie in Riechstoffmischungen für Weichspüler eingearbeitet werden.

Es wurde ferner gemäß einem weiteren Aspekt der Erfindung gefunden, dass sich 2-Isopropyl-5-methyloxepan der Formel **A** insbesondere in geringen Konzentrationen hervorragend zum Verstärken eines bereits vorhandenen Geruchs eignet, d.h. dass 2-Isopropyl-5-methyloxepan der Formel **A** insbesondere als so genannter Booster oder Enhancer fungieren kann; dies zeigt sich insbesondere bei Kompositionen mit blumigen Noten. Die verstärkende Wirkung übertrifft dabei deutlich den additiven Effekt, der gegebenenfalls dadurch verursacht wird, dass die Verbindung der Formel **A** in geringen Konzentrationen selbst einen blumigen Geruch vermittelt. Die Erfindung betrifft daher zudem die Verwendung von 2-Isopropyl-5-methyloxepan der Formel **A** als Booster (Verstärker; Enhancer) für andere Riechstoffe, insbesondere für blumige Riechstoffe.

Ferner eignen sich 2-Isopropyl-5-methyloxepan der Formel **A** bzw. Riechstoffmischungen umfassend 2-Isopropyl-5-methyloxepan der Formel **A** wegen ihrer ausgeprägten Diffusivität (Raumwirkung) besonders zur Einarbeitung in Produkte zur Raumluftverbesserung (vorzugsweise in flüssiger, gelartiger oder auf einem festen Träger aufgebrachter Form sowie Pumpsprays oder Aerosolsprays). Ein bevorzugter parfümierter Artikel ist deshalb eine Raumluftverbesserungs-Formulierung.

Für die Parfümierung von tensidhaltigen Formulierungen, wie zum Beispiel für Shampoos, Waschmitteln oder Weichspülern werden Riechstoffmischungen benötigt, die eine prägnante Kopfnote besitzen und gleichzeitig ein ausgeprägtes Blooming (Geruch aus einer wässrigen Tensidlösung) aufweisen sollten.

Die erfindungsgemäße Verbindung 2-Isopropyl-5-methyloxepan der Formel **A** besitzt eine prägnante Ylang-Ylang- und Patchouli-Kopfnote mit Aspekten von Indol und Hyazinthe, wobei auch interessante kresolige Nuancen auftreten. Gleichzeitig bewirkt der Einsatz der erfindungsgemäßen Verbindung **A** in tensidhaltigen wässigen Lösungen ein überraschendes Blooming, wobei insbesondere der über der tensidhaltigen wässrigen Lösung (z. B. einer Waschlauge) wahrgenommene Geruch anderer Riechstoffe in überraschender Weise überhöht wird.

Dementsprechend betrifft ein weiterer Aspekt der Erfindung die Verwendung von 2-Isopropyl-5-methyloxepan der Formel **A** zum Erhöhen des über einer tensidhaltigen wässrigen Lösung (z. B. einer Waschlauge) wahrgenommenen Geruches anderer Riechstoffe, d. h. zum Erhöhen des Blooming dieser anderen Riechstoffe.

Durch Verwendung des 2-Isopropyl-5-methyloxepans der Formal **A** lässt sich regelmäßig bereits in geringer Dosierung in einer resultierenden Riechstoffmischung (insbesondere Parfümkomposition) eine gewünschte prägnante Kopfnote erreichen, wobei in wässrigen, tensidhaltigen Formulierungen in geringen Dosierungen auch bereits ein ausgeprägte s Blooming und eine hohe Substantivität bewirkt wird.

Es hat sich gezeigt, dass das erfindungsgemäße 2-Isopropyl-5-methyloxepan der Formel **A** in wässrigen, tensidhaltigen Anwendungen / Formulierungen mit basischem pH-Wert ein besonders ausgeprägtes Blooming bewirkt, vorzugsweise liegt der pH-Wert der tensidhaltigen Formulierung dann im Bereich von pH 8 bis 13. Vorzugsweise ist in einer erfindungsgemäßen wässigen, tensidhaltigen Lösung dieser pH-Bereich eingestellt.

Die besonderen olfaktorischen Eigenschaften und weitere hervorragende stoffli-che Eigenschaften , wie Löslichkeit in üblichen kosmetischen Lösungsmitteln, Kompatibilität mit üblichen weiteren Bestandteilen derartiger Produkte etc., unterstreichen die besondere Eignung der erfindungsgemäßen Verbindung für die genannten Einsatzzwecke.

Die Herstellung von 2-Isopropyl-5-methyloxepan der Formel **A** erfolgt vorzugsweise ausgehend von 3,7-Dimethyloct-1-en-1,6-diol (I), welches aus der Photo- En-Reaktion von Citronellol mit Singulettsauerstoff erhalten werden kann (DE 1 137 730, US 5,892,059, siehe hierzu auch Beispiel 2, unten) durch Cyclisierung des Diols (I) zum einfach ungesättigten Oxepan (II) mit anschließender Reduktion, vorzugsweise katalytischer Hydrierung, wie das folgende Reaktionsschema exemplarisch verdeutlicht:

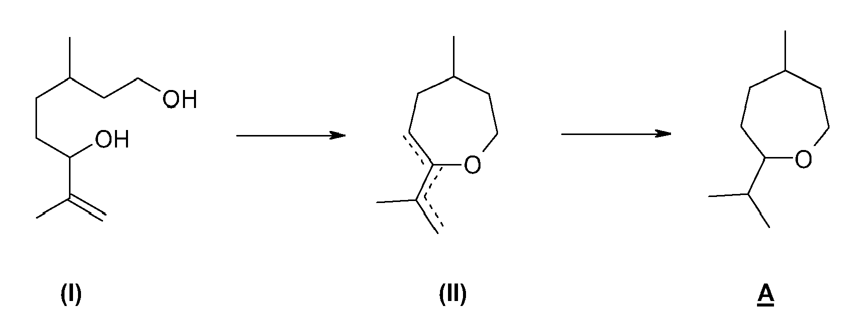

Die gestrichelte Linie in dem ungesättigten Oxepan (II) bedeutet dabei eine Doppelbindung in einer der drei angegebenen Positionen; das ungesättigte Oxepan (II) umfasst somit folgende Verbindungen:

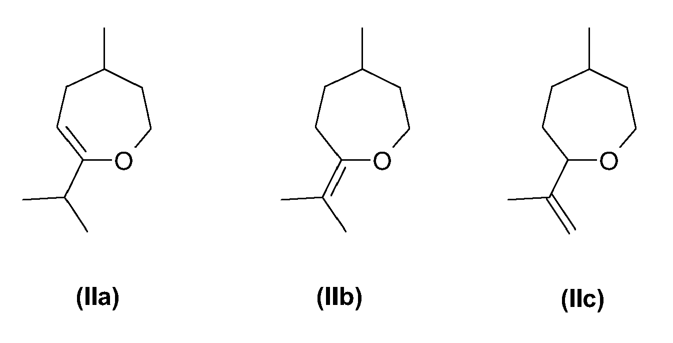

Die Cyclisierung vom Diol (I) zum Oxepan (II) erfolgt vorzugsweise in Gegenwart von dehydratisierend wirkenden Reagenzien, wie z. B. starken anorganischen Protonensäuren (z. B. Schwefelsäure) oder organischen Säuren (z. B. para-Toluolsulfonsäure). Daneben sind auch (vorzugsweise wasserfreie) LewisSäuren (z. B. Fe-(III)-chlorid, in Anlehnung an literaturbekannte Verfahren, vgl. Hisashi Yamamoto, Lewis Acids in Organic Chemistry, Wiley - VCH, Weinheim, New York, 2000) und anorganische Salze starker Säuren (z. B. Kupfersulfat) zur Unterstützung der Dehydratisierung geeignet. So gelingt beispielweise analog zu J. Org. Chem. 1980, 45, 917-919 die Cyclisierung vom Diol (I) zu dem ungesättigten Oxepan (II) in Gegenwart von wasserfreiem Cu-(II)-sulfat oder in Gegenwart einer Mischung von Cu-(II)-sulfat und para-Toluolsulfonsäure). Vorzugsweise wird dabei das Diol (I) in eine siedende Mischung von die Dehydratisierung unterstützendem Reagenz (Katalysator) und Toluol getropft.

Die Reduktion des ungesättigten Oxepan (II) zu dem erfindungsgemäßen 2-Isopropyl-5-methyloxepan der Formel **A** erfolgt vorzugsweise mittels Hydrierung. Die dabei verwendeten Wasserstoffdrücke liegen dabei vorzugsweise im Bereich von 5 bis 250 bar, vorzugsweise im Bereich von 20 bis 150 bar. Die Temperaturen bei der Hydrierung liegen vorzugsweise im Bereich von 30 bis 250 °C, bevorzugt im Bereich von 60 bis 150 °C, und hängen dabei im Wesentlichen vom verwendeten Katalysatorsystem ab. Aus der Literatur (P. N. Rylander, Catalytic Hydrogenation in Organic Synthesis, Academic Press, New York, 1979; Robert L. Augustine Heterogeneous Catalysis for the Synthetic Chemist, Marcel Dekker Verlag, 1995) sind eine Vielzahl homogener oder heterogener Katalysatorsysteme bekannt, mit denen die Hydrierung unter den beschriebenen Bedingungen durchgeführt werden kann. Die heterogenen Katalysatorsysteme bestehen beispielsweise aus Raney-Nickel oder aus Ruthenium, Platin oder Palladium, vorzugsweise auf Trägermaterialien. Als vorteilhafte Trägermaterialen seien Aktivkohle, Kohle, Aluminiumoxide, Metalloxide, Kieselgele, Zeolithe, Tone, Tongranulate oder amorphe Aluminiumsilicate genannt. Bevorzugter Hydrierkatalysatoren ist Nickel, besonders bevorzugt ist Raney-Nickel. Die Hydrierung kann mit oder ohne Lösungsmittel durchgeführt werden. Als Lösungsmittel können dabei z. B. gesättigte C₁-C₄ -Alkohole, vorzugsweise Methanol, eingesetzt werden.

2-Isopropyl-5-methyloxepan der Formel **A** wird vorzugsweise aus 5-Methylpropenyloxepan (entsprechend der Verbindung (IIc)) durch Hydrierung in Gegenwart von Raney-Nickel in Methanol bei bis zu 80 °C und 20 bar Druck Wasserstoffgas hergestellt.

Alternativ kann 2-Isopropyl-5-methyloxepan der Formel **A** durch Dehydratisierung und Cyclisierung von 3,7-Dimethyloctan-1,6-diol (III) in Gegenwart von dehydratisierenden Reagenzien, wie z. B. wasserfreiem Kupfer(II)-sulfat, hergestellt werden (in Anlehnung an literaturbekannte Verfahren, s.o.). 3,7-Dimethyloctan-1,6-diol (III) wiederum ist z. B. durch Hydrierung von 3,7-Dimethyloct-1-en-1,6-diol (I) nach gängigen Methoden (P. N. Rylander, Catalytic Hydrogenation in Organic Synthesis, Academic Press, New York, 1979; Robert L. Augustine Heterogeneous Catalysis for the Synthetic Chemist, Marcel Dekker Verlag, 1995) herstellbar.

Eine weitere Möglichkeit zur Herstellung von 2-Isopropyl-5-methyloxepan der Formel **A** besteht in der basischen Cyclisierung von Verbindungen der Formel (IV), welche eine geeignete Abgangsgruppe X aufweisen (X bedeutet dabei vorzugsweise Mesylat, Tosylat, Benzolsulfonat). Die Verbindungen der Formel (IV) können ausgehend von 3,7-Dimethyloctan-1,6-diol (III) nach literaturbekannten Verfahren zur Einführung von Schutzgruppen bzw. Abgangsgruppen erhalten werden.

Das nachfolgende Reaktionsschema veranschaulicht die Synthesewege, die von 3,7-Dimethyloct-1-en-1,6-diol (III) ausgehen:

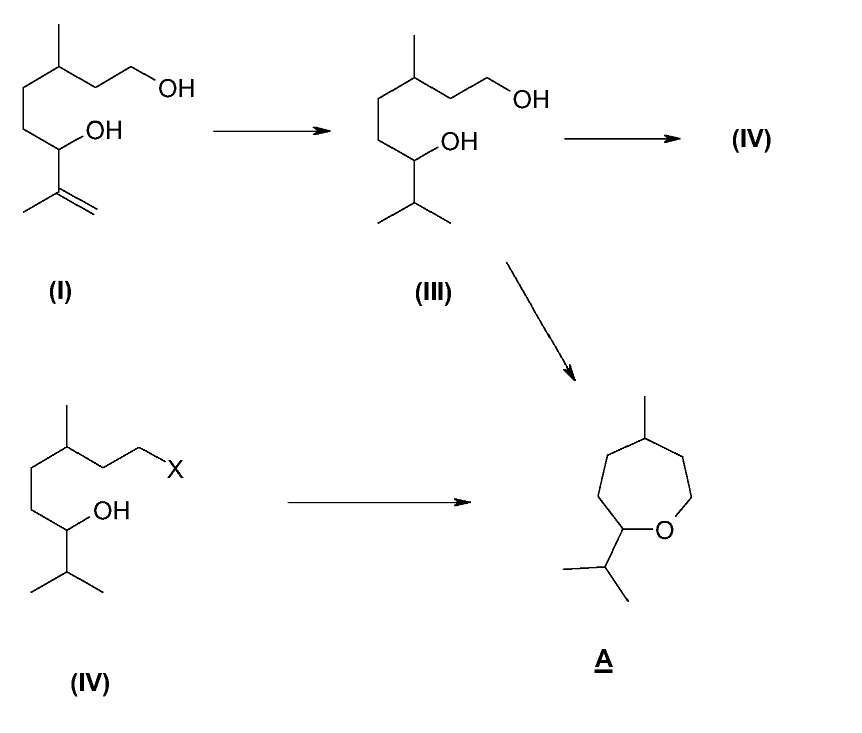

2-Isopropyl-5-methyloxepan der Formel **A** eignet sich - wie oben im Detail erläutert - insbesondere wegen seiner olfaktorischen Eigenschaften vorzüglich für den Einsatz in Riechstoffmischungen. Die Verbindung kann dabei als Einzelstoff oder mit einer Vielzahl weiterer Riechstoffe kombiniert in zahlreichen Produkten verwendet werden. Besonders vorteilhaft lässt sich die Verbindung mit anderen Riechstoffen in verschiedenen, unterschiedlichen Mengenverhältnissen zu neuartigen Parfümkompositionen (als Beispiele für spezielle Riechstoffmischungen) kombinieren.

Beispiele für Riechstoffe, mit denen die erfindungsgemäße Verbindung vorteilhaft kombiniert werden kann, finden sich z. B. in S. Arctander, Perfume and Flavor Chemicals, Vol. I und II, Montclair, N. J., 1969, Selbstverlag oder H. Surburg, J. Panten, Common Fragrance and Flavor Materials, 5rd. Ed., Wiley-VCH, 2006. Im einzelnen seien genannt:
Extrakte aus natürlichen Rohstoffen wie Etherische Öle, Concretes, Absolues, Resine, Resinoide, Balsame, Tinkturen wie z. B.
Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Maj o-ranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z. B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z. B. Hexanol; Octanol; 3-Octanol; 2,6-Dimethylheptanol; 2-Methyl-2-heptanol; 2-Methyl-2-octanol; (E)-2-Hexenol; (E)-und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z. B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z. B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z. B. 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril;
der Ester von aliphatischen Carbonsäuren wie z. B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Geranial; Neral; Citronel-Ial; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon ; 8-Mercaptomenthan-3-on ; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton ; Dihydronootkaton ; 4,6,8-Megastigmatrien-3-on; alpha-Sinensal ; beta-Sinensal ; acetyliertes Cedernholzöl (Methylcedrylketon);
der cyclischen Alkohole wie z. B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z. B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z. B. Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z. B. 4-tert-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z. B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z. B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z. B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z. B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z. B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z. B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1 H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z. B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z. B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z. B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z. B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z. B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

In Parfümöl-Kompositionen (= Riechstoffmischungen) beträgt die eingesetzte Menge des erfindungsgemäßen 2-Isopropyl-5-methyloxepans **A** vorzugsweise 0,0001 bis 70 Gew.-%, vorzugsweise 0,01 bis 50 Gew.-% und besonders bevorzugt 0,25 bis 25 Gew.-%, bezogen auf die Gesamtmenge der ParfümölKomposition.

Sofern 2-Isopropyl-5-methyloxepan der Formel **A** hauptsächlich eingesetzt wird, um einer Riechstoffkomposition (Aus)Strahlung, Abrundung und/oder Harmonie zu verleihen und/oder bestimmte Noten lediglich zu verstärken, insbesondere Noten der Richtungen blumig (wie z. B. Hyacinthe, Jasmin), orientalisch (wie z. B. Patchouli) und/oder maskulin, ist der Anteil des erfindungsgemäßen 2-Isopropyl-5-methyloxepans **A** allerdings vorzugsweise recht niedrig und liegt häufig im Bereich von 0,001 bis 2 Gew.-%, bevorzugt im Bereich von 0,01 bis 1 Gew.-%, bezogen auf die Gesamtmenge der Riechstoffmischung. Sofern innerhalb der bevorzugten Konzentrationsbereiche eine vergleichsweise niedrige Konzentration gewählt wird, kommt es in Abhängigkeit von den weiteren Komponenten der jeweiligen Komposition in manchen Fällen noch nicht zur Vermittlung der oben angegebenen Eigengeruchsnoten.

2-Isopropyl-5-methyloxepan der Formel **A** hat eine Geruchsschwelle in Luft von etwa 2,5 10⁻⁸ g/L Luft (entsprechend 25 ng/L).

2-Isopropyl-5-methyloxepan der Formel **A** enthaltende Riechstoffmischungen (Parfümöle) können in flüssiger Form, unverdünnt oder mit einem Lösungmittel verdünnt, für Parfümierungen eingesetzt werden. Geeignete Lösungsmittel sind z. B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Für manche Anwendungen ist es vorteilhaft, 2-Isopropyl-5-methyloxepan der Formel A enthaltende Riechstoffmischungen (Parfümöle) an einem Trägerstoff adsorbiert einzusetzen, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker oder Kunststoffe wie PVC, Polyvinylacetate oder Polyurethane sein.

Für andere Anwendungen ist es vorteilhaft, 2-Isopropyl-5-methyloxepan der Formel **A** enthaltende Riechstoffmischungen (Parfümöle) mikroverkapselt, sprühgetrocknet, als Einschluss-Komplex oder als Extrusions-Produkt einzusetzen und in dieser Form dem zu parfümierenden (Vor-)Produkt hinzufügen.

Die Eigenschaften derart modifizierter Riechstoffmischungen (Parfümöle) werden in manchen Fällen durch sogenanntes "Coaten" mit geeigneten Materialien im Hinblick auf eine gezieltere Duftfreisetzung weiter optimiert, wozu vorzugsweise wachsartige Kunststoffe wie z. B. Polyvinylalkohol verwendet werden.

Die Mikroverkapselung der Riechstoffmischungen (Parfümöle) kann beispielsweise durch das sogenannte Koazervationsverfahren mit Hilfe von Kapselmaterialien z. B. aus polyurethanartigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z. B. durch Eintragen von Dispersionen von dem Parfümöl und Cyclodextrinen oder Harnstoffderivaten in ein geeignetes Lösungsmittel, z. B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z. B. Isopropanol, erfolgen.

Das erfindungsgemäße 2-Isopropyl-5-methyloxepan der Formel A kann vorzugsweise mit einem oder mehreren der folgenden Zutatent kombiniert werden: Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Slilcone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

2-Isopropyl-5-methyloxepan der Formel **A** enthaltende Parfümöle (Riechstoffmischungen) können in konzentrierter Form, in Lösungen oder in sonstiger modifizierter Form verwendet werden für die Herstellung von erfindungsgemäßen parfümierten Artikeln wie z. B. Parfüm-Extraits, Eau de Parfums, Eau de Toilettes, Rasierwässer, Eau de Colognes, Pre-shave-Produkte, Splash-Colognes, parfümierten Erfrischungstüchern parfümierten sauren, alkalischen (bevorzugt) und neutralen Reinigungsmitteln und tensidhaltigen Formulierungen wie z. B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln, Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z. B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z. B. Hautcremes- und -lotionen, Gesichtscremes und -lotionen, Sonnenschutzcremes- und -lotionen, After-sun-cremes und -lotionen, Handcremes und -lotionen, Fußcremes und -lotionen, Enthaarungscremes und -lotionen, Aftershave-Cremes und -lotionen, Bräunungscremes und -lotionen, Haarpflegeprodukten wie z. B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z. B. Achselsprays, Roll-ons, Deosticks, Deocremes oder Produkten der dekorativen Kosmetik.

Nachfolgend wird die Erfindung anhand von Beispielen näher erläutert. Sofern nicht anders angegeben beziehen sich alle Angaben auf das Gewicht.

### Beispiel 1: Jasmin-Duftthema: Parfümkomposition (Z1) mit 2-Isopropyl-5-methyloxepan der Formel A im Vergleich mit Parfümkomposition (X1) ohne 2-Isopropyl-5-methyloxepan der Formel A

| | **X1** | **Z1** |
|---|---|---|
| | Gewichtsteile | Gewichtsteile |
| Acetessigsäureethylester | 2 | 2 |
| Aldehyd C14 (sogenannt) | 2 | 2 |
| Benzylacetat | 250 | 250 |
| Benzylbenzoat | 191 | 191 |
| Benzylpropionat | 2 | 2 |
| Eugenol | 15 | 15 |
| Methyl dihydrojasmonat | 150 | 150 |
| Alpha-Hexylzimtaldehyd | 200 | 200 |
| Indol, 10%ig in Dipropylenglycol | 8 | 8 |
| Indolen | 20 | 20 |
| Isojasmon | 10 | 10 |
| Jasmin absolue, ägyptisch | 5 | 5 |
| Kresol, para | 3 | 3 |
| Linalool | 80 | 80 |
| Maltol, 1%ig in Benzylbenzoat | 40 | 40 |
| Phenylethyldimethylcarbinol | 20 | 20 |
| Ylang Ylang-Öl Extra | 2 | 2 |
| 2-Isopropyl-5-methyloxepan der Formel **A**^{#} | | 2 |
| Summe: | 1000 | 1002 |

2-Isopropyl-5-methyloxepan der Formel **A**^{#} : racemisches Gemisch der Diastereomeren im Massenverhältnis 65 : 34 (vermutlich trans : cis).

### Geruchsbeschreibung der nicht erfindungsgemäßen Parfümkomposition X1: Jasmin.

Nach Meinung der Parfümeure wird in der Riechstoffkomposition Z1 durch die Anwesenheit von 2 Gewichtsteilen 2-Isopropyl-5-methyloxepan der Formel **A** im Vergleich mit der Basis-Riechstoff-Komposition X1 eine stärkere Hervorhebung (Wirkung als Booster, Enhancer) der Jasminnote, eine größere Diffusivität (Raumwirkung) und eine höhere Ausstrahlung erzielt; der Eindruck von Blumigkeit wird zudem erheblich verstärkt.

### Beispiel 2: Darstellung von 2-Isopropyl-5-methyloxepan A

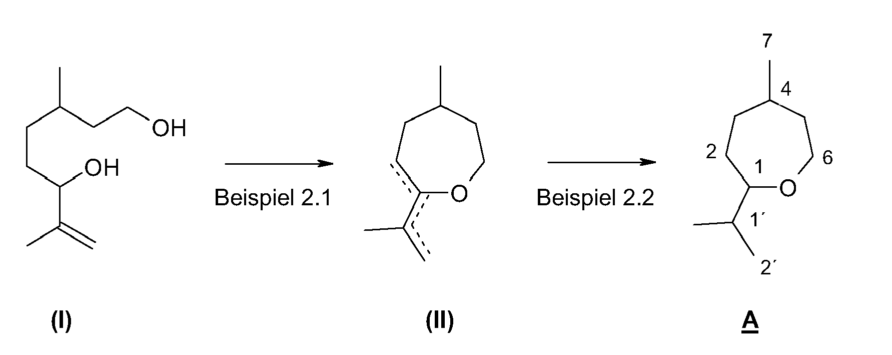

### Beispiel 2.1: Herstellung des ungesättigten Oxepans (II)

In 150 g Toluol werden 5,72 g Kupfersulfat (0,04 mol) und 1,72 g para-Toluolsulfonsäure (0,009 mol) vorgelegt. Die Suspension wird unter Verwendung eines Wasserabscheiders auf Rückflusstemperatur erhitzt. In die siedende Katalysatorsuspension werden dann 500 g des Diols (I) (2,32 mol) (Herstellung wie in US 5,892,059 beschrieben) derart eingetropft, dass die Reaktionsmischung weiter siedet. Nach vollständigem Umsatz wird das ungesättigte Oxepan (II) durch Destillation abgetrennt. Das ungesättigte Oxepan (II) wird in 42%iger Ausbeute in 75%iger Reinheit (GC-MS) durch Destillation erhalten und in die anschließende Hydrierung (Beispiel 2.2) eingesetzt.

### Beispiel 2.2: Hydrierung des ungesättigten Oxepans (II)

1394 g (5,97 mol) des ungesättigten Oxepans (II) (durch Vereinigen mehrerer Ansätze der ersten Stufe, Beispiel 2.1) werden zusammen mit 1500 g Methanol und 8,3 g Raney-Nickel (Bezugsquelle: Degussa) entsprechend etwa 0,6 Gew.-% bezogen auf die Menge des ungesättigten Oxepans (II) in einem 5L-Stahlautoklaven (Firma Hofer) vorgelegt und unter 20 bar Wasserstoffdruck hydriert. Nach vollständiger Umsetzung wird der Reaktor entspannt und das Rohprodukt durch Filtration über Talkum vom Katalysator abgetrennt.

Nach Abdestillieren des Methanols wird 2-Isopropyl-5-methyloxepan der Formel **A** als Gemisch der cis- und trans - Isomere mit einer Ausbeute von 81,4% und einem Gehalt von >99% (GC-MS) isoliert. Mittels Feindestillation (Füllkörperko-Ionne, Sulzer BX- Packung) werden Fraktionen der fast reinen cis- und trans - Isomere (Reinheit jeweils >95 Gew.-%) isoliert.
¹H-NMR (400 MHz, CDCl₃, TMS): 0,866 (C7H₃); 0,91 (C2'H₃); 1,225 (C4H); 1,433 (C3H₂); 1,635 (C2H₂); 1,672 (C5H₂); 3,190 (C1 H); 3,356 (C6H₂); 3,981 (C1'H)ppm
¹³C-NMR (400 MHz, CDCl₃, TMS): 18,60, 18,93 (C2'); 23,73 (C7); 33,46 (C1'); 33,65 (C4); 40,63 (C5, C3), 70,33 (C2, C6), 85,23 (C1) ppm MS (m/z, %): 156 (molpeak); 113; 95; 73; 69; 56; 43.

### Beispiel 3: Anwendungstechnische parfümistische Aspekte

2-Isopropyl-5-methyloxepan der Formel **A** wurde unter verschiedenen parfümistischen Gesichtpunkten untersucht; die Ergebnisse sind in der folgenden Tabelle zusammengefasst. Die Bewertung erfolgte hierbei jeweils gegen den Standard ω-Bromstyrol.

| | |
|---|---|
| | 2-Isopropyl-5-methyloxepan der Formel **A** |
| Impact | größer als Standard |
| Haftung | wie Standard |
| Geruchsschwelle | etwa um den Faktor 12 niedriger als Standard |
| Intensität | etwas schwächer als Standard |
| Diffusivität | größer als Standard |
| Blooming | wie Standard |

### Beispiel 4: Shampoo

Die Parfümölkomposition Z1 aus Beispiel 1 (enthaltend das erfindungsgemäße 2-Isopropyl-5-methyloxepan **A**) wurde in einer Dosierung von 0,5 Gewichtsteilen in eine Shampoo-Grundmasse folgender Zusammensetzung eingearbeitet:

| | Gewichtsteile |
|---|---|
| Natriumlaurylethersulfat (z. B. Texapon NSO, Fa. Cognis Deutschland GmbH) | 12 |
| Cocamidopropylbetain (z. B. Dehyton K, Fa. Cognis Deutschland GmbH) | 2 |
| Natriumchlorid | 1,4 |
| Citronensäure | 1,3 |
| Phenoxyethanol, Methyl-, Ethyl-, Butyl-, | |
| und Propylparaben | 0,5 |
| Wasser | 82,8 |
| Summe: | 100 |

Der pH-Wert der resultierenden, Z1 enthaltenden Shampoo-Grundmasse lag bei etwa 6. Aus ihr wurden 100 mL einer 20 Gew.%-igen wässrigen Shampoo-Lösung hergestellt. In dieser Shampoo-Lösung wurden 2 Haarsträhnchen gemeinsam für 2 Minuten gewaschen und anschließend 20 Sekunden unter fließendem handwarmem Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt. Beide Haarsträhnen zeigten im Vergleich mit Haarsträhnen, welche mit einem Vergleichs-Shampoo enthaltend einen gleichen Anteil der Basis-Riechstoff-Komposition X1 aus Beispiel 1 (ohne Verbindung **A**) gewaschen worden waren, eine stärkere Jasminnote, der Eindruck von Blumigkeit war verstärkt, wobei der Gesamteindruck als strahlender, abgerundeter und harmonischer empfunden wurde.

### Beispiel 5: Weichspüler

Die Parfümölkomposition Z1 aus Beispiel 1 (enthaltend das erfindungsgemäße 2-Isopropyl-5-methyloxepan **A**) wurde in einer Dosierung von 0,5 Gewichtsteilen in eine Weichspüler-Grundmasse folgender Zusammensetzung eingearbeitet:

| | Gewichtsteile |
|---|---|
| Quarternäres Ammoniummethosulfat (Esterquat), ca. 90% (z. B. Rewoquat WE 18, Fa. Witco Surfactants GmbH) | 5,5 |
| Alkyldimethylbenzylammoniumchlorid, ca. 50% (z. B. Preventol R50, Fa. Bayer AG) | 0,2 |
| Farblösung, ca. 1%-ig | 0,3 |
| Wasser | 94,0 |
| Summe: | 100 |

Der pH-Wert der resultierenden, Z1 enthaltenden Weichspüler-Grundmasse lag im Bereich 2-3. Hieraus wurde eine 1 Gew.%-ige wässrige Weichspülerlösung hergestellt. Zwei Stofflappen wurden mit 370 g der 1 %-igen wässrigen Weichspüler-Lösung in einer Linetest-Maschine im Weichspülprogramm 30 Minuten bei 20 °C gespült. Die Lappen wurden ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt. Beide Lappen zeigten im Vergleich mit Lappen, welche entsprechend mit einer Vergleichs-Weichspülerlösung enthaltend einen gleichen Anteil der Basis-Riechstoff-Komposition X1 aus Beispiel 1 (ohne Verbindung **A**) gewaschen worden waren, eine stärkere Hervorhebung der Jasminnote, der Eindruck von Blumigkeit war verstärkt, wobei der Gesamteindruck als strahlender, abgerundeter und harmonischer empfunden wurde.

### Beispiel 6: Parfümkomposition (P1) mit Patchouligeruch

| | P1 |
|---|---|
| | Gewichtsteile |
| Isobornylacetat | 5,5 |
| L-Borneol | 25,0 |
| Kampfer | 15,0 |
| Davanaöl | 1,0 |
| Nopol | 95,0 |
| Dimethylbenzylcarbinylacetat | 5,5 |
| Terpineol | 11,0 |
| Palmarosaöl | 6,0 |
| Caryophyllen | 20,0 |
| para tert.-Butylcyclohexanol | 32,5 |
| Cedernholzöl, Florida | 25,0 |
| Palisandin ¹⁾ (Cyclododecylmethylether) | 162,0 |
| Vetiveröl, Java | 5,5 |
| Gurjunbalsam, Siam | 102,5 |
| Sandel 80 ¹⁾ | 7,5 |
| Fixateur Bois | 35,0 |
| Elemi Resin EE | 36,5 |
| Opoponax Resin, entwachst | 5,0 |
| Karanal²) [2-(2,4-Dimethyl-3-cyclohexenyl)-5-methyl-5-(1-methylpropyl)-1,3-dioxan] | 2,5 |
| Globalid ¹⁾ (11(12)-Pentadecen-15-olide) | 2,0 |
| Patchouliöl | 100,0 |
| Isolongifolanol | 50,0 |
| 2-Isopropyl-5-methyloxepan der Formel **A**^{#} | 40,0 |
| Summe: | 790,0 |

| | |
|---|---|
| 1) Handelsnamen der Fa. Symrise 2) Handelsname der Fa. Quest | |

2-Isopropyl-5-methyloxepan der Formel **A**^{#}: racemisches Gemisch der Diastereomeren im Massenverhältnis 50 : 50.

### Beispiel 7: Waschpulver

Die Parfümölkomposition P1 aus Beispiel 6 (enthaltend das erfindungsgemäße 2-Isopropyl-5-methyloxepan **A**) wurde in einer Dosierung von 0,4 Gewichtsteilen in eine Waschpulver-Grundmasse der folgenden Rezeptur eingearbeitet:

| | Gewichtsteile |
|---|---|
| Lineares Na-Alkylbenzolsulfonat | 8,8 |
| Ethoxylierter Fettalkohol C12-18 (7 EO) | 4,7 |
| Na-Seife | 3,2 |
| Entschäumer | |
| DOW CORNING(R) 2-4248S | |
| POWDERED ANTIFOAM, | |
| Silikonöl auf Zeolith als Trägermaterial | 3,9 |
| Zeolith 4A | 28,3 |
| Na-Carbonat | 11,6 |
| Na-Salz eines Copolymers aus Acryl-und Maleinsäure (Sokalan CP5) | 2,4 |
| Na-Silikat | 3,0 |
| Carboxymethylcellulose | 1,2 |
| Dequest 2066 ([[(Phosphonomethyl)imino]bis[(ethylennitrilo)bis (methylen)]]tetrakis-phosphonsäure, Natriumsalz) | 2,8 |
| Optischer Aufheller | 0,2 |
| Na-Sulfat | 6,5 |
| Protease | 0,4 |
| Natriumperborattetrahydrat | 22,0 |
| TAED | 1,0 |
| **Summe:** | 100 |

Hieraus wurde eine 1 %-ige wässrige Waschpulverlauge hergestellt. Zwei Stofflappen wurden mit 370 g der 1 %-igen wässrigen Waschpulverlauge (der pH-Wert der Waschpulverlauge liegt deutlich im basischen Bereich) in einer Linetest-Maschine im Hauptwaschgang 45 Minuten bei 60 °C gewaschen. Die Lappen wurden zunächst 5 Minuten mit kaltem Wasser gespült, ausgewrungen und anschließend 20 Sekunden geschleudert. Ein Lappen wurde nass eingeschweißt und der andere zum Trocknen aufgehängt.

### Beispiel 8: Air-freshener in Gelform

5 g Accurel (poröses Homo-Polypropylen Pulver mit 75% Hohlraumanteil, ein Produkt der Akzo Nobel Faser AG, Obernburg, Deutschland) werden mit 15 g der Parfümölkomposition Z1 aus Beispiel 1 durch Mischen der beiden Bestandteile unter Vakuum beladen. Das resultierende Pulver wird anschließend bei Normaldruck mit 4,5 g Wasser verrührt (Mix 1). In einem separaten Gefäß werden 2,5 g Carragheen, 0,3 g Chloracetamid und 0,5 g Calciumchlorid-Dihydrat in 62 g Wasser unter Erwärmen bis auf etwa 75 °C gelöst. In diese Lösung wird unter Rühren Mix 1 eingebracht und homogenisiert. Die resultierende, noch warme Mischung wird in die gewünschte Form (Kugeln, Halbkugeln, Kissen, Zylinder, Quader, Würfel, Muscheln oder ähnliche) gegossen. Nach dem Abkühlen auf etwa 20°C werden Raumerfrischer in Gelform erhalten, deren Beladung an Parfümölkomposition Z1 bei etwa 20 Gew.-% liegt.

## Patentansprüche

1. 2-Isopropyl-5-methyloxepan der Formel **A:**

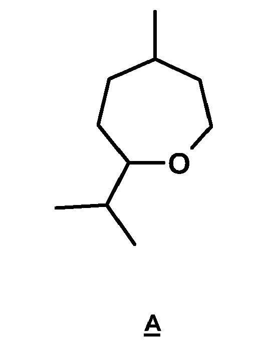

2. Verwendung von 2-Isopropyl-5-methyloxepan der Formel **A** als Riechstoff.

3. Verwendung nach Anspruch 2 als Riechstoff mit dem Geruchsprofil Indol, Kresol, Patchouli, Hyazinthe, Ylang-Ylang, animalisch.

4. Verwendung nach Anspruch 2 oder 3 als Riechstoff mit Patchouli- und/oder Ylang-Ylang-Kopfnote.

5. Verfahren zum Vermitteln, Modifizieren und/oder Verstärken einer, mehrerer oder sämtlicher der Geruchsnoten Indol, Kresol, Patchouli, Hyazinthe, Ylang-Ylang, animalisch oder Blume in einer Riechstoffmischung,vorzugsweise zumindest einer, mehrerer oder sämtlicher der Geruchsnoten Indol, Patchouli, Hyazinthe, Ylang-Ylang oder Blume, mit folgendem Schritt:
- Mischen der sonstigen Bestandteile der Riechstoffmischung mit einer sensorisch wirksamen Menge an 2-Isopropyl-5-methyloxepan der Formel **A.**

6. Verfahren nach Anspruch 5, zum Vermitteln, Modifizieren und/oder Verstärken einer Patchouli-Kopfnote.

7. Verfahren nach Anspruch 5, zum Vermitteln, Modifizieren und/oder Verstärken der Geruchnsote Blume, mit folgendem Schritt:
- Mischen der sonstigen Bestandteile der Riechstoffmischung mit einer sensorisch wirksamen Menge an 2-Isopropyl-5-methyloxepan der Formel **A**, so dass das 2-Isopropyl-5-methyloxepan der Formel **A** in der resultierenden Riechstoffmischung in hoher Verdünnung vorliegt.

8. Riechstoffmischung oder parfümierter Artikel, umfassend 2-Isopropyl-5-methyloxepan der Formel **A** in einer sensorisch wirksamen Menge.

9. Riechstoffmischung oder parfümierter Artikel nach Anspruch 8, wobei die sensorisch wirksame Menge so gewählt ist, dass das 2-Isopropyl-5-methyloxepan der Formel **A** eine, mehrere oder sämtliche der Geruchsnoten Indol, Kresol, Patchouli, Hyazinthe, Ylang-Ylang, animalisch oder Blume, vorzugsweise zumindest eine, mehrere oder sämtliche der Geruchsnoten Indol, Patchouli, Hyazinthe, Ylang-Ylang oder Blume, vermittelt, modifiziert oder verstärkt.

10. Riechstoffmischung oder parfümierter Artikel nach Anspruch 8 oder 9, wobei die vorhandene Menge an 2-Isopropyl-5-methyloxepan der Formel **A** eine Patchouli- und/oder Ylang-Ylang-Kopfnote vermittelt, modifiziert oder verstärkt.

11. Riechstoffmischung oder parfümierter Artikel nach Anspruch 8 oder 9, wobei das vorhandene 2-Isopropyl-5-methyloxepan der Formel **A** so hoch verdünnt ist, dass es einen blumigen Geruch vermittelt, modifiziert oder verstärkt.

12. Riechstoffmischung nach einem der Ansprüche 8 bis 11, umfassend eine Menge an 2-Isopropyl-5-Methyloxepan der Formel **A** im Bereich von 0,0001 bis 70 Gew.-%, vorzugsweise 0,1 bis 50 Gew.-% und besonders bevorzugt 0,25 bis 25 Gew.-%, bezogen auf die Gesamtmenge der Riechstoffmischung.

13. Parfümierter Artikel nach einem der Ansprüche 8 bis 11, wobei der Artikel eine tensidhaltige oder eine Raumluftverbesserungs-Formulierung ist.

14. Verfahren zur Herstellung einer Riechstoffmischung, mit folgendem Schritt:
- Vermischen einer sensorisch wirksamen Menge von 2-Isopropyl-5-methyloxepan der Formel **A** mit weiteren Bestandteilen einer Riechstoffmischung.

15. Verfahren nach Anspruch 14, zur Herstellung einer Riechstoffmischung nach einem der Ansprüche 8 bis 12.

16. Verfahren zur Herstellung von 2-Isopropyl-5-methyloxepan der Formel **A**, mit folgendem Schritt:
- Herstellen oder Bereitstellen einer, mehrerer oder sämtlicher Verbindungen der Formeln (IIa), (IIb) und (IIc) 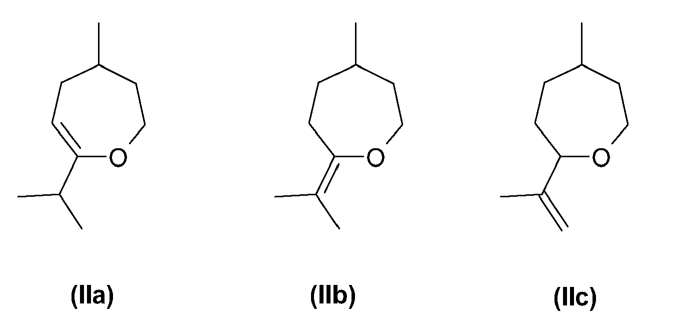
- Hydrieren der Verbindung bzw. Verbindungen der Formeln (IIa), (IIb) und (IIc) zum 2-Isopropyl-5-methyloxepan der Formel **A.**
